# EUROPÄISCHE PATENTSCHRIFT

(11) **EP 3 834 776 B1**
(45) Veröffentlichungstag und Bekanntmachung des Hinweises auf die Patenterteilung: **21.08.2024**
(21) Anmeldenummer: 20209251.6
(22) Anmeldetag: 23.11.2020
(51) Int. Cl.: A61F 2/16

(54) **INTRAOKULARLINSE**
INTRAOCULAR LENS
LENTILLE INTRAOCULAIRE

(30) Priorität: 12.12.2019 DE 102019134178
(43) Veröffentlichungstag der Anmeldung: 16.06.2021
(73) Patentinhaber: Carl Zeiss Meditec AG, 07745 Jena (DE)
(72) Erfinder: BENSAID, Nicolas, 10719 Berlin (DE)
(74) Vertreter: PATERIS Patentanwälte PartmbB

(56) Entgegenhaltungen:
- US-A- 4 343 050
- US-A- 5 571 177
- US-A1- 2003 135 271

## Beschreibung

Die Erfindung betrifft eine Intraokularlinse.

Bei der Kataraktbehandlung eines Auges wird herkömmlich ein Schnitt in die Hornhaut des Auges eingebracht, der so groß ist, dass eine Kanüle durch den Schnitt in das Auge eingeführt werden kann. Nachdem der Schnitt in die Hornhaut eingebracht wurde, wird die Linse des Auges mittels Phakoemulsifikation zerkleinert und anschließend aus dem Kapselsack des Auges abgesaugt. Danach wird eine Intraokularlinse mittels eines Injektors in den Kapselsack eingesetzt. Die Intraokularlinse weist einen Optikkörper und eine Haptik auf, wobei mittels der Haptik der Optikkörper in dem Kapselsack fixiert wird.

Die Haptik hat die Aufgabe, den Optikkörper möglichst mittig in dem Auge zu halten, um eine möglichst gute Abbildung auf der Netzhaut des Auges zu erzeugen. Zudem soll der Optikkörper möglichst positionsstabil in dem Kapselsack festgelegt sein. Außerdem hat die Haptik die Aufgabe, den Optikkörper davon abzuhalten, eine Rotation um seine optische Achse durchzuführen. Dies ist besonders relevant, wenn es sich bei dem Optikkörper um einen torischen Optikkörper handelt, mittels dessen eine Hornhautverkrümmung korrigiert werden soll, weil der torische Optikkörper, in dem Fall, dass er mit einer falschen Orientierung in dem Kapselsack angeordnet ist, zu einem Abbildungsfehler auf der Netzhaut führt.

In dem Fall, dass die Intraokularlinse zu klein für den Kapselsack ist, ist es schwierig, die Intraokularlinse positionsstabil in dem Kapselsack zu halten. Sollte die Intraokularlinse zu groß für den Kapselsack sein, besteht die Gefahr einer Verletzung des Kapselsacks durch die Intraokularlinse und zudem ist es schwierig, die Intraokularlinse in dem Kapselsack zu rotieren, um bei dem torischen Optikkörper diesen mit der richtigen Orientierung in dem Kapselsack anzuordnen.

Aufgabe der Erfindung ist es daher, eine Intraokularlinse zu schaffen, mit der die vorgenannten Probleme gelöst werden können.

US 2003/0135271 A1 offenbart eine Intraokularlinse mit einer oder mehreren zerbrechlichen Strukturen, die zwischen den Haptiken und der Optik der Intraokularlinse gebildet ist.

Die erste erfindungsgemäße Intraokularlinse weist einen Optikkörper, mindestens zwei Haptiken und für mindestens zwei der Haptiken jeweils einen Satz auf, der eine Mehrzahl an Seilen aufweist, die jeweils an dem Optikkörper und an der zu dem Satz zugehörigen Haptik befestigt sind und eine Durchtrennungsreihenfolge haben, wobei jede der mindestens zwei der Haptiken jeweils einen komprimierten Zustand, einen teilweise komprimierten Zustand und einen unkomprimierten Zustand hat, wobei für jede der mindestens zwei der Haptiken und dem zu der jeweiligen Haptik zugehörigen Satz gilt, dass in dem komprimierten Zustand das erste Seil der Durchtrennungsreihenfolge eingerichtet ist, die Haptik in Richtung zu dem Optikkörper hin zu deformieren, wodurch das erste Seil der Durchtrennungsreihenfolge unter einer Zugspannung steht und die verbliebenen Seile spannungsfrei sind, und dass die Haptik durch nacheinander erfolgendes Durchtrennen der Seile in der Durchtrennungsreihenfolge zuerst in den teilweise unkomprimierten Zustand, in dem dasjenige der Seile, das nicht durchtrennt ist und die niedrigste Ordnungszahl der Durchtrennungsreihenfolge hat, eingerichtet ist, die Haptik in Richtung zu dem Optikkörper hin zu deformieren und somit unter einer Zugspannung steht, und die verbliebenen der Seile, die nicht durchtrennt sind, spannungsfrei sind, und schließlich in den unkomprimierten Zustand gebracht werden kann, in dem alle die Seile durchtrennt sind.

Die zweite erfindungsgemäße Intraokularlinse weist einen Optikkörper, mindestens zwei Haptiken und für mindestens zwei der Haptiken jeweils einen Satz auf, der eine Mehrzahl an Federn aufweist, die jeweils an dem Optikkörper und an der zu dem Satz zugehörigen Haptik befestigt sind und eine Durchtrennungsreihenfolge haben, wobei jede der mindestens zwei der Haptiken jeweils einen komprimierten Zustand, einen teilweise komprimierten Zustand und einen unkomprimierten Zustand hat, wobei für jede der mindestens zwei der Haptiken und dem zu der jeweiligen Haptik zugehörigen Satz gilt, dass in dem komprimierten Zustand die Gesamtheit der Federn eingerichtet ist, die Haptik in Richtung zu dem Optikkörper hin zu deformieren, wodurch zumindest die erste Feder der Durchtrennungsreihenfolge aus ihrer Ruheposition verlängert ist, und dass die Haptik durch nacheinander erfolgendes Durchtrennen der Federn in der Durchtrennungsreihenfolge zuerst in den teilweise komprimierten Zustand, in dem die Gesamtheit der nicht durchtrennten Federn eingerichtet ist, die Haptik in Richtung zu dem Optikkörper hin zu deformieren, und zumindest diejenige der Federn, die nicht durchtrennt ist und die niedrigste Ordnungszahl der Durchtrennungsreihenfolge hat, aus ihrer Ruheposition verlängert ist, und schließlich in den unkomprimierten Zustand gebracht werden kann, in dem alle der Federn durchtrennt sind.

Die dritte erfindungsgemäße Intraokularlinse weist einen Optikkörper, mindestens zwei Haptiken und für mindestens zwei der Haptiken jeweils einen Satz auf, der eine Mehrzahl an Federn aufweist, die jeweils an dem Optikkörper und an der zu dem Satz zugehörigen Haptik befestigt sind, wobei jede der mindestens zwei der Haptiken jeweils einen komprimierten Zustand, einen teilweise komprimierten Zustand und einen unkomprimierten Zustand hat, wobei für jede der mindestens zwei der Haptiken und dem zu der jeweiligen Haptik zugehörigen Satz gilt, dass in dem komprimierten Zustand alle der Federn in einem komprimierten Federzustand sind, und dass die Haptik durch nacheinander erfolgendes Bringen der Federn in einen unkomprimierten Federzustand zuerst in den teilweise komprimierten Zustand, in dem zumindest eine der Federn in dem komprimierten Federzustand und zumindest eine der Federn in dem unkomprimierten Federzustand sich befinden, und schließlich in den unkomprimierten Zustand gebracht werden kann, in dem alle der Federn sich in dem unkomprimierten Zustand befinden.

Die Begriffe "komprimierter Zustand", "teilweise komprimierter Zustand" und "unkomprimierter Zustand" beziehen sich dabei auf die Intraokularlinse, die sich außerhalb des Kapselsacks befindet und sich ohne eine Begrenzung von dem komprimierten Zustand in den unkomprimierten Zustand bewegen kann. Die Seile können jeweils eine Faser oder jeweils mehrere Fasern aufweisen. In der Ruheposition liegen bei der zweiten erfindungsgemäßen Intraokularlinse in der Axialrichtung der Feder keine Kräfte an der Feder an. Die Intraokularlinse ist dazu vorgesehen, in einen Kapselsack eines Auges eingesetzt zu werden, wenn die Seile beziehungsweise die Federn nicht durchtrennt sind beziehungsweise alle die Federn sich in dem komprimierten Federzustand befinden und sich die Intraokularlinse damit in dem komprimierten Zustand befindet. Zum Einsetzen in den Kapselsack wird die Intraokularlinse gefaltet und in dem gefalteten Zustand in den Kapselsack injiziert, in dem sich die Intraokularlinse entfaltet. Weil in dem komprimierten Zustand die Haptik mittels der Seile beziehungsweise der Federn in Richtung zu dem Optikkörper hin deformiert ist, kann sich die Haptik dabei nicht unkontrolliert entfalten, wodurch die Gefahr einer Verletzung des Kapselsacks gering ist. Zudem nimmt die Intraokularlinse nur wenig Raum ein und ist, wenn überhaupt, nur geringfügig mittels der Haptiken an dem Kapselsack befestigt. Dadurch ist es einfach möglich, die Position der Intraokularlinse zu verändern. Dies ist beispielsweise relevant, um die Intraokularlinse für eine gute Abbildung mittig in dem Kapselsack anzuordnen. Zudem ist es einfach möglich, die Orientierung der Intraokularlinse zu verändern. Dadurch können Abbildungsfehler vermieden werden in dem Fall, dass es sich bei dem Optikkörper um einen torischen Optikkörper handelt.

Für die erste und zweite erfindungsgemäße Intraokularlinse können die Seile beziehungsweise die Federn nun mittels eines Messers oder einer Schere, die via einen kleinen Schnitt in der Hornhaut in den Kapselsack eingeführt werden, oder mittels eines Lasers durchtrennt werden, um die Intraokularlinse in den teilweise komprimierten Zustand oder in den unkomprimierten Zustand zu bringen und dadurch zu vergrößern. Um die Intraokularlinse möglichst gleichmäßig zu vergrößern, können bei allen den Sätzen zuerst diejenigen Seile beziehungsweise Federn mit der gleichen Ordnungszahl durchtrennt werden, bevor die Seile beziehungsweise die Federn mit der nächst höheren Ordnungszahl durchtrennt werden. Dadurch kann gewährleistet werden, dass die Intraokularlinse beim Durchtrennen der Seile beziehungsweise der Federn möglichst mittig in dem Kapselsack angeordnet bleibt. Bei der dritten erfindungsgemäßen Intraokularlinse erfolgt dies, indem die Federn nacheinander in den unkomprimierten Federzustand gebracht werden. Zudem ist es möglich, eine Anpassung der Größe der Intraokularlinse an den Kapselsack vorzunehmen, indem nicht alle der Seile beziehungsweise der Federn durchtrennt werden beziehungsweise nicht alle der Federn in den unkomprimierten Federzustand gebracht werden. Dadurch kann die Gefahr einer Verletzung des Kapselsacks durch die Haptiken vermindert werden. Dasjenige der Seile, das nicht durchtrennt ist und die niedrigste Ordnungszahl der Durchtrennungsreihenfolge hat, kann unter einer Zugspannung stehen. Alle die Seile, die nicht durchtrennt sind, stehen unter keiner Druckspannung.

Für die erste erfindungsgemäße Intraokularlinse ist es erfindungsgemäß, dass für jedes der Seile gilt, dass das Seil an einem Haptikbefestigungspunkt an der Haptik und an einem Optikkörperbefestigungspunkt an dem Optikkörper befestigt ist, wobei der Haptikbefestigungspunkt und der Optikkörperbefestigungspunkt in derselben Ebene liegen, deren Normale mit der optischen Achse des Optikkörpers zusammenfällt. Für die zweite erfindungsgemäße Intraokularlinse ist es bevorzugt, dass für jede der Federn gilt, dass die Feder an einem Haptikbefestigungspunkt an der Haptik und an einem Optikkörperbefestigungspunkt an dem Optikkörper befestigt ist, wobei der Haptikbefestigungspunkt und der Optikkörperbefestigungspunkt in derselben Ebene liegen, deren Normale mit der optischen Achse des Optikkörpers zusammenfällt. Dadurch kann vermieden werden, dass sich die Intraokularlinse im komprimierten Zustand oder im teilweise komprimierten Zustand wölbt.

Für die erste erfindungsgemäße Intraokularlinse ist es bevorzugt, dass jeder der Sätze aus zwei der Seile besteht oder dass jeder der Sätze drei der Seile aufweist oder daraus besteht oder dass jeder der Sätze vier der Seile aufweist oder daraus besteht. Für die zweite erfindungsgemäße Intraokularlinse ist es bevorzugt, dass jeder der Sätze aus zwei der Federn besteht oder dass jeder der Sätze drei der Federn aufweist oder daraus besteht oder wobei jeder der Sätze vier der Federn aufweist oder daraus besteht.

Für die erste erfindungsgemäße Intraokularlinse ist es bevorzugt, dass für jeden der Sätze gilt, dass alle die Seile eine unterschiedliche Farbe haben. Für die zweite erfindungsgemäße Intraokularlinse ist es bevorzugt, dass für jeden der Sätze gilt, dass alle die Federn eine unterschiedliche Farbe haben. Durch die unterschiedliche Farbe kann ein Operateur einfach die Durchtrennungsreihenfolge erkennen.

Die Seile gemäß der ersten erfindungsgemäßen Intraokularlinse sind bevorzugt in der Durchtrennungsreihenfolge immer länger ausgeführt. Dadurch ist es beispielsweise möglich, dass alle die Seile eines der Sätze an einem identischen Haptikbefestigungspunkt an der Haptik und an einem identischen Optikkörperbefestigungspunkt an dem Optikkörper befestigt sind. Dadurch kann die Anzahl der Optikkörperbefestigungspunkte minimiert werden. Weil jeder der Optikkörperbefestigungspunkte die Abbildung durch den Optikkörper beeinträchtigen kann, kann durch das Minimieren der Anzahl der Optikkörperbefestigungspunkte auch eine Beeinträchtigung der abbildenden Funktion des Optikkörpers minimiert werden.

Für die zweite erfindungsgemäße Intraokularlinse ist es bevorzugt, dass jede der mindestens zwei Haptiken ein außenliegendes Haptiklängsende aufweist und für jeden der Sätze gilt, dass der Abstand der Federn von dem außenliegenden Haptiklängsende in der Durchtrennungsreihenfolge immer länger ausgeführt ist. Dadurch kann verhindert werden, dass die Federn eines der Sätze in Kontakt kommen und sich dadurch verfangen.

Für die erste erfindungsgemäße Intraokularlinse ist es bevorzugt, dass die Seile eingerichtet sind, sich nach Einsetzen der Intraokularlinse in einen Kapselsack eines Auges zu zersetzen. Für die zweite erfindungsgemäße Intraokularlinse ist es bevorzugt, dass die Federn eingerichtet sind, sich nach Einsetzen der Intraokularlinse in einen Kapselsack eines Auges zu zersetzen. Dadurch kann erreicht werden, dass kein überflüssiges Material in dem Kapselsack verbleibt.

Für die erste erfindungsgemäße Intraokularlinse ist es bevorzugt, dass die Seile eingerichtet sind, sich nach Einsetzen der Intraokularlinse in einen Kapselsack eines Auges und nur dann zu zersetzen, wenn sie durchtrennt wurden. Für die zweite erfindungsgemäße Intraokularlinse ist es bevorzugt, dass die Federn eingerichtet sind, sich nach Einsetzen der Intraokularlinse in einen Kapselsack eines Auges und nur dann zu zersetzen, wenn sie durchtrennt wurden. Dadurch ist es möglich, die Größe der Intraokularlinse an die Größe des Kapselsacks anzupassen, indem nur diejenigen der Seile/Federn durchtrennt werden, die dazu notwendig sind, und gleichzeitig zu vermeiden, dass überflüssiges Material in dem Kapselsack verbleibt.

Für die dritte erfindungsgemäße Intraokularlinse ist es bevorzugt, dass die Federn ein Formgedächtnismaterial aufweisen, so dass durch ein Aufwärmen des Formgedächtnismaterials jede der Federn in den unkomprimierten Federzustand bringbar ist. Das Aufwärmen kann beispielsweise mittels eines Lasers erfolgen.

Für die dritte erfindungsgemäße Intraokularlinse ist es alternativ bevorzugt, dass die Intraokularlinse für jede der Federn jeweils eine Haltevorrichtung aufweist, die eingerichtet ist, die zu der Haltevorrichtung zugehörige Feder in dem komprimierten Federzustand zu halten, wobei durch ein Öffnen der Haltevorrichtung die zu der Haltevorrichtung zugehörige Feder in den unkomprimierten Federzustand bringbar ist. Die Haltevorrichtung kann beispielsweise zwei Platten, die an den beiden Längsenden der Feder angeordnet sind, und ein Haltevorrichtungsseil aufweisen, das an beiden Platten befestigt ist. Das Öffnen der Haltevorrichtung kann durch ein Durchtrennen des Haltevorrichtungsseils erfolgen, beispielsweise mittels eines Messers, einer Pinzette oder eines Lasers.

Die mindestens zwei der Haptiken sind für alle erfindungsgemäßen Intraokularlinsen bevorzugt C-förmig oder J-förmig. Besonders bevorzugt sind alle die Haptiken C-förmig oder J-förmig.

Für alle erfindungsgemäßen Intraokularlinsen ist es bevorzugt, dass die Intraokularlinse drei oder vier der Haptiken und für jede der Haptiken einen der Sätze aufweist. Besonders bevorzugt weist die Intraokularlinse nur zwei, nur drei oder nur vier der Haptiken und für jede der Haptiken einen der Sätze auf.

Der Optikkörper ist für alle erfindungsgemäßen Intraokularlinsen bevorzugt ein torischer Optikkörper. Zusätzlich oder alternativ ist denkbar, dass es sich bei dem Optikkörper um einen monofokalen oder multifokalen Optikkörper handelt. Zudem ist zusätzlich oder alternativ denkbar, dass der Optikkörper eine erweiterte Schärfentiefe hat (englisch: enhanced depth of focus, EDoF).

Im Folgenden wird anhand der beigefügten schematischen Zeichnung die Erfindung näher erläutert.
Figur 1 zeigt eine erste Ausführungsform einer ersten erfindungsgemäßen Intraokularlinse in einem komprimierten Zustand.
Figur 2 zeigt die erste Ausführungsform der ersten erfindungsgemäßen Intraokularlinse in einem teilweise komprimierten Zustand.
Figur 3 zeigt eine zweite Ausführungsform der ersten erfindungsgemäßen Intraokularlinse in einem teilweise komprimierten Zustand.
Figur 4 zeigt eine zweite erfindungsgemäße Intraokularlinse in einem komprimierten Zustand.

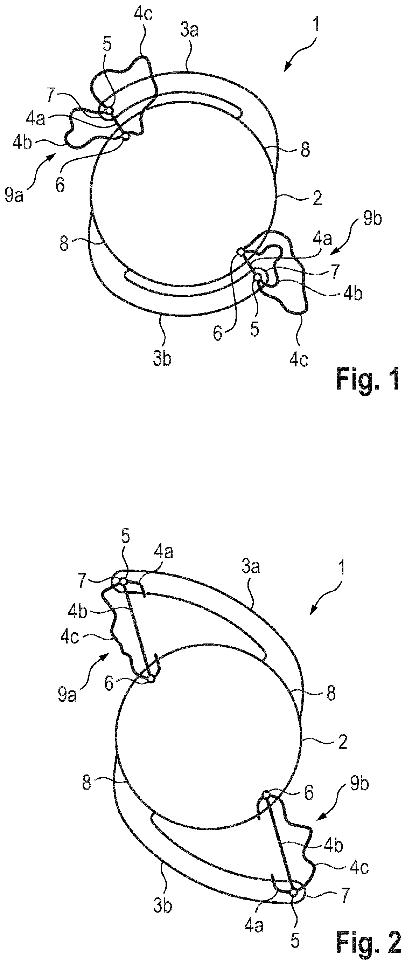

Wie es aus Figuren 1 bis 3 ersichtlich ist, weist eine erste erfindungsgemäße Intraokularlinse 1 einen Optikkörper 2, mindestens zwei Haptiken 3a, 3b und für mindestens zwei der Haptiken 3a, 3b jeweils einen Satz 9a, 9b auf, der eine Mehrzahl an Seilen 4a, 4b, 4c aufweist, die jeweils an dem Optikkörper 2 und an der zu dem Satz zugehörigen Haptik 3a, 3b befestigt sind und eine Durchtrennungsreihenfolge haben. Jede der mindestens zwei der Haptiken 3a, 3b hat jeweils einen komprimierten Zustand, einen teilweise komprimierten Zustand und einen unkomprimierten Zustand. Für jede der mindestens zwei der Haptiken 3a, 3b und dem zu der jeweiligen Haptik 3a, 3b zugehörigen Satz 9a, 9b gilt, dass in dem komprimierten Zustand das erste Seil 4a der Durchtrennungsreihenfolge eingerichtet ist, die Haptik 3a, 3b in Richtung zu dem Optikkörper 2 hin zu deformieren, wodurch das erste Seil 4a der Durchtrennungsreihenfolge unter einer Zugspannung steht und die verbliebenen Seile 4b, 4c spannungsfrei sind, und dass die Haptik 3a, 3b durch nacheinander erfolgendes Durchtrennen der Seile 4a, 4b, 4c in der Durchtrennungsreihenfolge zuerst in den teilweise komprimierten Zustand, in dem dasjenige der Seile 4b, das nicht durchtrennt ist und die niedrigste Ordnungszahl der Durchtrennungsreihenfolge hat, eingerichtet ist, die Haptik 3a, 3b in Richtung zu dem Optikkörper 2 hin zu deformieren und somit unter einer Zugspannung steht, und die verbliebenen der Seile 4b, 4c, die nicht durchtrennt sind, spannungsfrei sind, und schließlich in den unkomprimierten Zustand gebracht werden kann, in dem alle die Seile 4a, 4b, 4c durchtrennt sind. Jede der Haptiken 3a, 3b kann ein außenliegendes Haptiklängsende 7 und ein innenliegendes Haptiklängsende 8 aufweisen, das an dem Optikkörper 2 befestigt ist.

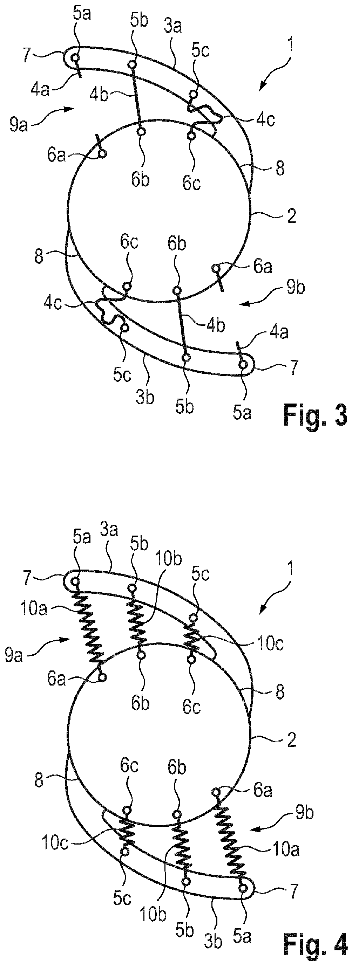

Figuren 1 bis 3 zeigen, dass für jede der mindestens zwei der Haptiken 3a, 3b und dem zu der jeweiligen Haptik 3a zugehörigen Satz 9a, 9b gelten kann, dass die Seile 4a, 4b, 4c an ihren ersten Längsenden an der zu den Seilen 4a, 4b, 4c zugehörigen Haptik 3a, 3b und an ihren zweiten Längsenden an dem Optikkörper 2 befestigt sind. Figuren 1 und 2 zeigen, dass gemäß einer ersten Ausführungsform der ersten erfindungsgemäßen Intraokularlinse 1 jede der mindestens zwei der Haptiken 3a, 3b einen Haptikbefestigungspunkt 5 aufweisen kann und der Optikkörper 2 für jeden der mindestens zwei der Haptiken 3a, 3b einen Optikkörperbefestigungspunkt 6 aufweisen kann, wobei bei jedem der Sätze 9a, 9b jedes der Seile 4a, 4b, 4c an dem zu dem Satz 9a, 9b zugehörigen Haptikbefestigungspunkt 5 und an dem zu dem Satz 9a, 9b zugehörigen Optikkörperbefestigungspunkt 6 befestigt ist. Figur 3 zeigt, dass gemäß einer zweiten Ausführungsform der ersten erfindungsgemäßen Intraokularlinse 1 für jede der mindestens zwei der Haptiken 3a, 3b und dem zu der Haptik 3a, 3b zugehörigen Satz 9a, 9b gelten kann, dass die Haptik 3a, 3b für jedes der Seile 4a, 4b, 4c einen jeweiligen Haptikbefestigungspunkt 5a, 5b, 5c aufweist und der Optikkörper 2 für jedes der Seile 4a, 4b, 4c einen jeweiligen Optikkörperbefestigungspunkt 6a, 6b, 6c aufweist und dass jedes der Seile 4a, 4b, 4c an einem anderen der Haptikbefestigungspunkte 5a, 5b, 5c und an einem anderen der Optikkörperbefestigungspunkte 6a, 6b, 6c befestigt ist.

Figur 1 zeigt den komprimierten Zustand für alle die Haptiken 3a, 3b, wobei erkennbar ist, dass für alle die Haptiken 3a, 3b das erste Seil 4a unter einer Zugspannung steht und alle verbliebenen der Seile 4b, 4c spannungsfrei sind. Figur 2 zeigt den teilweise komprimierten Zustand für alle die Haptiken 3a, 3b, wobei in dem in Figur 2 dargestellten teilweise komprimierten Zustand für alle die Haptiken 3a, 3b das erste Seil 4a durchtrennt ist und alle verbliebenen der Seile 4b, 4c nicht durchtrennt sind. In Figur 2 ist zudem zu erkennen, dass das zweite Seil 4b unter einer Zugspannung steht und das verbliebene Seil 3c nicht durchtrennt und spannungsfrei ist.

Wie es aus Figuren 1 bis 3 ersichtlich ist, kann jeder der Sätze 9a, 9b aus drei der Seilen 4a, 4b, 4c bestehen. Alternativ ist denkbar, dass jeder der Sätze 9a, 9b aus zwei der Seilen 4a, 4b besteht oder dass jeder der Sätze 9a, 9b drei der Seile 4a, 4b, 4c aufweist oder dass jeder der Sätze 9a, 9b vier der Seile 4a, 4b, 4c aufweist oder daraus besteht.

Es ist denkbar, dass für jeden der Sätze 9a, 9b gilt, dass alle die Seile 4a, 4b, 4c eine unterschiedliche Farbe haben. Zudem ist es aus Figuren 1 bis 3 ersichtlich, dass die Seile 4a, 4b, 4c in der Durchtrennungsreihenfolge immer länger ausgeführt sein können. Es ist denkbar, dass die Seile 4a, 4b, 4c eingerichtet sind, sich nach Einsetzen der Intraokularlinse 1 in einen Kapselsack eines Auges zu zersetzen. Alternativ ist denkbar, dass die Seile 4a, 4b, 4c eingerichtet sind, sich nach Einsetzen der Intraokularlinse 1 in einen Kapselsack eines Auges und nur dann zu zersetzen, wenn sie durchtrennt wurden. Die Seile 4a, 4b, 4c können jeweils eine Faser oder jeweils mehrere Fasern aufweisen. Um zu vermeiden, dass sich die Intraokularlinse 1 im komprimierten Zustand oder im teilweise komprimierten Zustand zumindest einer der Haptiken 3a, 3b wölbt, kann für jedes der Seile 4a, 4b, 4c gelten, dass das Seil 4a, 4b, 4c an einem Haptikbefestigungspunkt 5, 5a, 5b, 5c an der Haptik 3a, 3b und an einem Optikkörperbefestigungspunkt 6, 6a, 6b, 6c an dem Optikkörper 2 befestigt ist, wobei der Haptikbefestigungspunkt 5, 5a, 5b, 5c und der Optikkörperbefestigungspunkt 6, 6a, 6b, 6c in derselben Ebene liegen, deren Normale mit der optischen Achse des Optikkörpers 2 zusammenfällt.

Wie es aus Figur 4 ersichtlich ist, weist eine zweite erfindungsgemäße Intraokularlinse 1 einen Optikkörper 2, mindestens zwei Haptiken 3a, 3b und für mindestens zwei der Haptiken 3a, 3b jeweils einen Satz 9a, 9b auf, der eine Mehrzahl an Federn 10a, 10b, 10c aufweist, die jeweils an dem Optikkörper 2 und an der zu dem Satz 9a, 9b zugehörigen Haptik 3a, 3b befestigt sind und eine Durchtrennungsreihenfolge haben. Jede der mindestens zwei der Haptiken 3a, 3b hat jeweils einen komprimierten Zustand, einen teilweise komprimierten Zustand und einen unkomprimierten Zustand. Für jede der mindestens zwei der Haptiken 3a, 3b und dem zu der jeweiligen Haptik 3a, 3b zugehörigen Satz 9a, 9b gilt, dass in dem komprimierten Zustand die Gesamtheit der Federn 10a, 10b, 10c eingerichtet ist, die Haptik 3a, 3b in Richtung zu dem Optikkörper 2 hin zu deformieren, wodurch zumindest die erste Feder 10a der Durchtrennungsreihenfolge aus ihrer Ruheposition verlängert ist, und dass die Haptik 3a, 3b durch nacheinander erfolgendes Durchtrennen der Federn 10a, 10b, 10c in der Durchtrennungsreihenfolge zuerst in den teilweise unkomprimierten Zustand, in dem die Gesamtheit der nicht durchtrennten Federn 10b, 10c eingerichtet ist, die Haptik 3a, 3b in Richtung zu dem Optikkörper 2 hin zu deformieren, und zumindest diejenige der Federn 10b, 10c, die nicht durchtrennt ist und die niedrigste Ordnungszahl der Durchtrennungsreihenfolge hat, aus ihrer Ruheposition verlängert ist, und schließlich in den unkomprimierten Zustand gebracht werden kann, in dem alle die Federn 10a, 10b, 10c durchtrennt sind. Jede der Haptiken 3a, 3b kann ein außenliegendes Haptiklängsende 7 und ein innenliegendes Haptiklängsende 8 aufweisen, das an dem Optikkörper 2 befestigt ist. In der Ruheposition liegen in der Axialrichtung der Feder 10a, 10b, 10c keine Kräfte an der Feder 10a, 10b, 10c an. In Figur 4 ist bei allen den Haptiken 3a, 3b der komprimierte Zustand dargestellt.

Figur 4 zeigt, dass jeder der Sätze 9a, 9b aus drei der Federn 10a, 10b, 10c bestehen kann. Alternativ ist denkbar, dass jeder der Sätze 9a, 9 aus zwei der Federn 10a, 10b besteht oder dass jeder der Sätze 9a, 9b drei der Federn 10a, 10b, 10c aufweist oder dass jeder der Sätze 9a, 9b vier der Federn 10a, 10b, 10c aufweist oder daraus besteht.

Für jeden der Sätze 9a, 9b kann gelten, dass alle die Federn 10a, 10b, 10c eine unterschiedliche Farbe haben. Zudem ist aus Figur 4 ersichtlich, dass jede der mindestens zwei der Haptiken 3a, 3b ein außenliegendes Haptiklängsende 7 und ein innenliegendes Haptiklängsende 8 aufweisen kann und für jeden der Sätze 9a, 9b gelten kann, dass der Abstand der Federn 10a, 10b, 10c von dem außenliegenden Haptiklängsende 7 in der Durchtrennungsreihenfolge immer länger ausgeführt ist. Es ist denkbar, dass die Federn 10a, 10b, 10c eingerichtet sind, sich nach Einsetzen der Intraokularlinse 1 in einen Kapselsack eines Auges zu zersetzen. Außerdem ist denkbar, dass die Federn 10a, 10b, 10c eingerichtet sind, sich nach Einsetzen der Intraokularlinse 1 in einen Kapselsack eines Auges und nur dann zu zersetzen, wenn sie durchtrennt wurden. Um zu vermeiden, dass sich die Intraokularlinse 1 im komprimierten Zustand oder im teilweise komprimierten Zustand zumindest einer der Haptiken 3a, 3b wölbt, kann für jede der Federn 10a, 10b, 10c gelten, dass das Seil 4a, 4b, 4c an einem Haptikbefestigungspunkt 5, 5a, 5b, 5c an der Haptik 3a, 3b und an einem Optikkörperbefestigungspunkt 6, 6a, 6b, 6c an dem Optikkörper 2 befestigt ist, wobei der Haptikbefestigungspunkt 5, 5a, 5b, 5c und der Optikkörperbefestigungspunkt 6, 6a, 6b, 6c in derselben Ebene liegen, deren Normale mit der optischen Achse des Optikkörpers 2 zusammenfällt.

Für beide erfindungsgemäßen Intraokularlinsen beziehen sich die Begriffe "komprimierter Zustand", "teilweise komprimierter Zustand" und "unkomprimierter Zustand" auf die Intraokularlinse, die sich außerhalb des Kapselsacks befindet und sich ohne eine Begrenzung von dem komprimierten Zustand in den unkomprimierten Zustand bewegen kann. Für beide erfindungsgemäßen Intraokularlinsen 1 können die mindestens zwei der Haptiken 3a, 3b beispielsweise C-förmig oder J-förmig sein. Zudem ist es für beide erfindungsgemäßen Intraokularlinsen 1 denkbar, dass die Intraokularlinse 1 drei oder vier der Haptiken 3a, 3b und für jede der Haptiken 3a, 3b einen der Sätze 9a, 9b aufweist, insbesondere weist die Intraokularlinse 1 nur zwei, nur drei oder nur vier der Haptiken 3a, 3b und für jede der Haptiken 3a, 3b einen der Sätze 9a, 9b auf. Zudem ist denkbar, dass der Optikkörper 2 ein torischer Optikkörper ist.

### Bezugszeichenliste

- 1: Intraokularlinse
- 2: Optikkörper
- 3a: erste Haptik
- 3b: zweite Haptik
- 4a: erstes Seil
- 4b: zweites Seil
- 4c: drittes Seil
- 5: Haptikbefestigungspunkt
- 5a: erster Haptikbefestigungspunkt
- 5b: zweiter Haptikbefestigungspunkt
- 5c: dritter Haptikbefestigungspunkt
- 6: Optikkörperbefestigungspunkt
- 6a: erster Optikkörperbefestigungspunkt
- 6b: zweiter Optikkörperbefestigungspunkt
- 6c: dritter Optikkörperbefestigungspunkt
- 7: außenliegendes Haptiklängsende
- 8: innenliegendes Haptiklängsende
- 9a: erster Satz
- 9b: zweiter Satz
- 10a: erste Feder
- 10b: zweite Feder
- 10c: dritte Feder

## Patentansprüche

1. Intraokularlinse mit einem Optikkörper (2), mindestens zwei Haptiken (3a, 3b) und für mindestens zwei der Haptiken (3a, 3b) jeweils einem Satz (9a, 9b), der eine Mehrzahl an Seilen (4a, 4b, 4c) aufweist, die jeweils an dem Optikkörper (2) und an der zu dem Satz zugehörigen Haptik (3a, 3b) befestigt sind und eine Durchtrennungsreihenfolge haben, wobei jede der mindestens zwei der Haptiken (3a, 3b) jeweils einen komprimierten Zustand, einen teilweise komprimierten Zustand und einen unkomprimierten Zustand hat, wobei für jede der mindestens zwei der Haptiken (3a, 3b) und dem zu der jeweiligen Haptik (3a) zugehörigen Satz (9a, 9b) gilt, dass in dem komprimierten Zustand das erste Seil (4a) der Durchtrennungsreihenfolge eingerichtet ist, die Haptik (3a, 3b) in Richtung zu dem Optikkörper (2) hin zu deformieren, wodurch das erste Seil (4a) der Durchtrennungsreihenfolge unter einer Zugspannung steht und die verbliebenen Seile (4b, 4c) spannungsfrei sind, und dass die Haptik (3a, 3b) durch nacheinander erfolgendes Durchtrennen der Seile (4a, 4b, 4c) in der Durchtrennungsreihenfolge zuerst in den teilweise komprimierten Zustand, in dem dasjenige der Seile (4b), das nicht durchtrennt ist und die niedrigste Ordnungszahl der Durchtrennungsreihenfolge hat, eingerichtet ist, die Haptik (3a, 3b) in Richtung zu dem Optikkörper (2) hin zu deformieren und somit unter einer Zugspannung steht, und die verbliebenen der Seile (4b, 4c), die nicht durchtrennt sind, spannungsfrei sind, und schließlich in den unkomprimierten Zustand gebracht werden kann, in dem alle die Seile (4a, 4b, 4c) durchtrennt sind, wobei für jedes der Seile (4a, 4b, 4c) gilt, dass das Seil (4a, 4b, 4c) an einem Haptikbefestigungspunkt (5, 5a, 5b, 5c) an der Haptik (3a, 3b) und an einem Optikkörperbefestigungspunkt (6, 6a, 6b, 6c) an dem Optikkörper (2) befestigt ist, wobei der Haptikbefestigungspunkt (5, 5a, 5b, 5c) und der Optikkörperbefestigungspunkt (6, 6a, 6b, 6c) in derselben Ebene liegen, deren Normale mit der optischen Achse des Optikkörpers (2) zusammenfällt.

2. Intraokularlinse gemäß Anspruch 1, wobei jeder der Sätze (9a, 9b) aus zwei der Seilen (4a, 4b) besteht oder wobei jeder der Sätze (9a, 9b) drei der Seile (4a, 4b, 4c) aufweist oder daraus besteht oder wobei jeder der Sätze (9a, 9b) vier der Seile (4a, 4b, 4c) aufweist oder daraus besteht.

3. Intraokularlinse gemäß Anspruch 1 oder 2, wobei für jeden der Sätze (9a, 9b) gilt, dass alle die Seile (4a, 4b, 4c) eine unterschiedliche Farbe haben.

4. Intraokularlinse gemäß einem der Ansprüche 1 bis 3, wobei die Seile (4a, 4b, 4c) in der Durchtrennungsreihenfolge immer länger ausgeführt sind.

5. Intraokularlinse mit einem Optikkörper (2), mindestens zwei Haptiken (3a, 3b) und für mindestens zwei der Haptiken (3a, 3b) jeweils einem Satz (9a, 9b), der eine Mehrzahl an Federn (10a, 10b, 10c) aufweist, die jeweils an dem Optikkörper (2) und an der zu dem Satz (9a, 9b) zugehörigen Haptik (3a, 3b) befestigt sind und eine Durchtrennungsreihenfolge haben, wobei jede der mindestens zwei der Haptiken (3a, 3b) jeweils einen komprimierten Zustand, einen teilweise komprimierten Zustand und einen unkomprimierten Zustand hat, wobei für jede der mindestens zwei der Haptiken (3a, 3b) und dem zu der jeweiligen Haptik (3a, 3b) zugehörigen Satz (9a, 9b) gilt, dass in dem komprimierten Zustand die Gesamtheit der Federn (10a, 10b, 10c) eingerichtet ist, die Haptik (3a, 3b) in Richtung zu dem Optikkörper (2) hin zu deformieren, wodurch zumindest die erste Feder (10a) der Durchtrennungsreihenfolge aus ihrer Ruheposition verlängert ist, und dass die Haptik (3a, 3b) durch nacheinander erfolgendes Durchtrennen der Federn (10a, 10b, 10c) in der Durchtrennungsreihenfolge zuerst in den teilweise komprimierten Zustand, in dem die Gesamtheit der nicht durchtrennten Federn (10b, 10c) eingerichtet ist, die Haptik (3a, 3b) in Richtung zu dem Optikkörper (2) hin zu deformieren, und zumindest diejenige der Federn (10b, 10c), die nicht durchtrennt ist und die niedrigste Ordnungszahl der Durchtrennungsreihenfolge hat, aus ihrer Ruheposition verlängert ist, und schließlich in den unkomprimierten Zustand gebracht werden kann, in dem alle die Federn (10a, 10b, 10c) durchtrennt sind.

6. Intraokularlinse mit einem Optikkörper (2), mindestens zwei Haptiken (3a, 3b) und für mindestens zwei der Haptiken (3a, 3b) jeweils einem Satz (9a, 9b), der eine Mehrzahl an Federn (10a, 10b, 10c) aufweist, die jeweils an dem Optikkörper (2) und an der zu dem Satz (9a, 9b) zugehörigen Haptik (3a, 3b) befestigt sind, wobei jede der mindestens zwei der Haptiken (3a, 3b) jeweils einen komprimierten Zustand, einen teilweise komprimierten Zustand und einen unkomprimierten Zustand hat, wobei für jede der mindestens zwei der Haptiken (3a, 3b) und dem zu der jeweiligen Haptik (3a, 3b) zugehörigen Satz (9a, 9b) gilt, dass in dem komprimierten Zustand alle der Federn (10a, 10b, 10c) in einem komprimierten Federzustand sind, und dass die Haptik (3a, 3b) durch nacheinander erfolgendes Bringen der Federn (10a, 10b, 10c) in einen unkomprimierten Federzustand zuerst in den teilweise komprimierten Zustand, in dem zumindest eine der Federn (10a, 10b, 10c) in dem komprimierten Federzustand und zumindest eine der Federn (10a, 10b, 10c) in dem unkomprimierten Federzustand sich befinden, und schließlich in den unkomprimierten Zustand gebracht werden kann, in dem alle der Federn (10a, 10b, 10c) sich in dem unkomprimierten Zustand befinden.

7. Intraokularlinse gemäß Anspruch 6, wobei die Federn (10a, 10b, 10c) ein Formgedächtnismaterial aufweisen, so dass durch ein Aufwärmen des Formgedächtnismaterials jede der Federn (10a, 10b, 10c) in den unkomprimierten Federzustand bringbar ist, oder wobei die Intraokularlinse (1) für jede der Federn (10a, 10b, 10c) jeweils eine Haltevorrichtung aufweist, die eingerichtet ist, die zu der Haltevorrichtung zugehörige Feder (10a, 10b, 10c) in dem komprimierten Federzustand zu halten, wobei durch ein Öffnen der Haltevorrichtung die zu der Haltevorrichtung zugehörige Feder (10a, 10b, 10c) in den unkomprimierten Federzustand bringbar ist.

8. Intraokularlinse gemäß einem der Ansprüche 1 bis 7, wobei die mindestens zwei der Haptiken (3a, 3b) C-förmig oder J-förmig sind.

9. Intraokularlinse gemäß einem der Ansprüche 1 bis 8, wobei die Intraokularlinse (1) drei oder vier der Haptiken (3a, 3b) und für jede der Haptiken (3a, 3b) einen der Sätze (9a, 9b) aufweist, insbesondere weist die Intraokularlinse (1) nur zwei, nur drei oder nur vier der Haptiken (3a, 3b) und für jede der Haptiken (3a, 3b) einen der Sätze (9a, 9b) auf.

10. Intraokularlinse gemäß einem der Ansprüche 1 bis 9, wobei der Optikkörper (2) ein torischer Optikkörper ist.

## Claims

1. Intraocular lens comprising an optical body (2), at least two haptic elements (3a, 3b) and for at least two of the haptic elements (3a, 3b) in each case a set (9a, 9b) having a plurality of ropes (4a, 4b, 4c), which are secured in each case to the optical body (2) and to the haptic element (3a, 3b) associated with the set and have a severing order, wherein each of the at least two of the haptic elements (3a, 3b) has in each case a compressed state, a partly compressed state and an uncompressed state, wherein for each of the at least two of the haptic elements (3a, 3b) and the set (9a, 9b) associated with the respective haptic element (3a) it holds true that in the compressed state the first rope (4a) of the severing order is configured to deform the haptic element (3a, 3b) in a direction toward the optical body (2), as a result of which the first rope (4a) of the severing order is under a tensile stress and the rest of the ropes (4b, 4c) are stress-free, and that by means of the ropes (4a, 4b, 4c) being severed successively in the severing order, the haptic element (3a, 3b) can be brought firstly into the partly compressed state, in which that one of the ropes (4b) which is not severed and has the lowest ordinal number of the severing order is configured to deform the haptic element (3a, 3b) in a direction toward the optical body (2) and is thus under a tensile stress, and the rest of the ropes (4b, 4c) which are not severed are stress-free, and finally into the uncompressed state, in which all the ropes (4a, 4b, 4c) are severed, wherein for each of the ropes (4a, 4b, 4c) it holds true that the rope (4a, 4b, 4c) is secured to the haptic element (3a, 3b) at a haptic element securing point (5, 5a, 5b, 5c) and is secured to the optical body (2) at an optical body securing point (6, 6a, 6b, 6c), wherein the haptic element securing point (5, 5a, 5b, 5c) and the optical body securing point (6, 6a, 6b, 6c) lie in the same plane, the normal to which coincides with the optical axis of the optical body (2).

2. Intraocular lens according to Claim 1, wherein each of the sets (9a, 9b) consists of two of the ropes (4a, 4b) or wherein each of the sets (9a, 9b) comprises or consists of three of the ropes (4a, 4b, 4c) or wherein each of the sets (9a, 9b) comprises or consists of four of the ropes (4a, 4b, 4c).

3. Intraocular lens according to Claim 1 or 2, wherein for each of the sets (9a, 9b) it holds true that all the ropes (4a, 4b, 4c) have a different colour.

4. Intraocular lens according to any of Claims 1 to 3, wherein the ropes (4a, 4b, 4c) are embodied as longer and longer in the severing order.

5. Intraocular lens comprising an optical body (2), at least two haptic elements (3a, 3b) and for at least two of the haptic elements (3a, 3b) in each case a set (9a, 9b) having a plurality of springs (10a, 10b, 10c), which are secured in each case to the optical body (2) and to the haptic element (3a, 3b) associated with the set (9a, 9b) and have a severing order, wherein each of the at least two of the haptic elements (3a, 3b) has in each case a compressed state, a partly compressed state and an uncompressed state, wherein for each of the at least two of the haptic elements (3a, 3b) and the set (9a, 9b) associated with the respective haptic element (3a, 3b) it holds true that in the compressed state the totality of the springs (10a, 10b, 10c) is configured to deform the haptic element (3a, 3b) in a direction toward the optical body (2), as a result of which at least the first spring (10a) of the severing order is lengthened from its rest position, and that by means of the springs (10a, 10b, 10c) being successively severed in the severing order, the haptic element (3a, 3b) can be brought firstly into the partly compressed state, in which the totality of the non-severed springs (10b, 10c) is configured to deform the haptic element (3a, 3b) in a direction toward the optical body (2), and at least that one of the springs (10b, 10c) which is not severed and has the lowest ordinal number of the severing order is lengthened from its rest position, and finally into the uncompressed state, in which all the springs (10a, 10b, 10c) are severed.

6. Intraocular lens comprising an optical body (2), at least two haptic elements (3a, 3b) and for at least two of the haptic elements (3a, 3b) in each case a set (9a, 9b) having a plurality of springs (10a, 10b, 10c), which are secured in each case to the optical body (2) and to the haptic element (3a, 3b) associated with the set (9a, 9b), wherein each of the at least two of the haptic elements (3a, 3b) has in each case a compressed state, a partly compressed state and an uncompressed state, wherein for each of the at least two of the haptic elements (3a, 3b) and the set (9a, 9b) associated with the respective haptic element (3a, 3b) it holds true that in the compressed state all of the springs (10a, 10b, 10c) are in a compressed spring state, and that by means of the springs (10a, 10b, 10c) being successively brought into an uncompressed spring state, the haptic element (3a, 3b) can be brought firstly into the partly compressed state, in which at least one of the springs (10a, 10b, 10c) is in the compressed spring state and at least one of the springs (10a, 10b, 10c) is in the uncompressed spring state, and finally into the uncompressed state, in which all of the springs (10a, 10b, 10c) are in the uncompressed state.

7. Intraocular lens according to Claim 6, wherein the springs (10a, 10b, 10c) comprise a shape memory material, such that each of the springs (10a, 10b, 10c) is able to be brought into the uncompressed spring state by means of the shape memory material being heated, or wherein the intraocular lens (1) comprises for each of the springs (10a, 10b, 10c) in each case a holding device configured to hold the spring (10a, 10b, 10c) associated with the holding device in the compressed spring state, wherein the spring (10a, 10b, 10c) associated with the holding device is able to be brought into the uncompressed spring state by means of the holding device being opened.

8. Intraocular lens according to any of Claims 1 to 7, wherein the at least two of the haptic elements (3a, 3b) are C-shaped or J-shaped.

9. Intraocular lens according to any of Claims 1 to 8, wherein the intraocular lens (1) comprises three or four of the haptic elements (3a, 3b) and one of the sets (9a, 9b) for each of the haptic elements (3a, 3b), in particular the intraocular lens (1) comprises only two, only three, or only four of the haptic elements (3a, 3b) and one of the sets (9a, 9b) for each of the haptic elements (3a, 3b).

10. Intraocular lens according to any of Claims 1 to 9, wherein the optical body (2) is a toric optical body.

## Revendications

1. Lentille intraoculaire avec un corps optique (2), au moins deux haptiques (3a, 3b) et, pour au moins deux des haptiques (3a, 3b), respectivement un ensemble (9a, 9b) qui présente une pluralité de câbles (4a, 4b, 4c), qui sont respectivement fixés au corps optique (2) et à l'haptique (3a, 3b) associé à l'ensemble, et ont une séquence de sectionnement, chacun des au moins deux haptiques (3a, 3b) ayant respectivement un état comprimé, un état partiellement comprimé et un état non comprimé ; pour chacun des au moins deux haptiques (3a, 3b) et l'ensemble (9a, 9b) associé à l'haptique respectif (3a), à l'état comprimé, le premier câble (4a) de la séquence de sectionnement étant adapté pour déformer l'haptique (3a, 3b) en direction du corps optique (2), moyennant quoi le premier câble (4a) de la séquence de sectionnement est soumis à une contrainte de traction et les câbles restants (4b, 4c) sont exempts de contrainte, et l'haptique (3a, 3b) pouvant être amené par sectionnement successif des câbles (4a, 4b, 4c) dans la séquence de sectionnement, d'abord à l'état partiellement comprimé, dans lequel celui des câbles (4b) qui n'est pas sectionné et qui a le numéro d'ordre le plus bas de la séquence de sectionnement est adapté pour déformer l'haptique (3a, 3b) en direction du corps optique (2) et est donc soumis à une contrainte de traction, et les câbles restants parmi les câbles (4b, 4c) qui ne sont pas sectionnés sont exempts de contrainte, et enfin à l'état non comprimé, dans lequel tous les câbles (4a, 4b, 4c) sont sectionnés ; pour chacun des câbles (4a, 4b, 4c), le câble (4a, 4b, 4c) étant fixé à un point de fixation d'haptique (5, 5a, 5b, 5c) à l'haptique (3a, 3b) et à un point de fixation de corps optique (6, 6a, 6b, 6c) au corps optique (2), le point de fixation d'haptique (5, 5a, 5b, 5c) et le point de fixation de corps optique (6, 6a, 6b, 6c) étant situés dans le même plan dont la normale coïncide avec l'axe optique du corps optique (2).

2. Lentille intraoculaire selon la revendication 1, dans laquelle chacun des ensembles (9a, 9b) est constitué de deux des câbles (4a, 4b) ou dans laquelle chacun des ensembles (9a, 9b) présente ou est constitué de trois des câbles (4a, 4b, 4c) ou dans laquelle chacun des ensembles (9a, 9b) présente ou est constitué de quatre des câbles (4a, 4b, 4c).

3. Lentille intraoculaire selon la revendication 1 ou 2, dans laquelle, pour chacun des ensembles (9a, 9b), tous les câbles (4a, 4b, 4c) ont une couleur différente.

4. Lentille intraoculaire selon l'une quelconque des revendications 1 à 3, dans laquelle les câbles (4a, 4b, 4c) sont toujours réalisés plus longs dans la séquence de sectionnement.

5. Lentille intraoculaire avec un corps optique (2), au moins deux haptiques (3a, 3b) et, pour au moins deux des haptiques (3a, 3b), respectivement un ensemble (9a, 9b) qui présente une pluralité de ressorts (10a, 10b, 10c) qui sont fixés respectivement au corps optique (2) et à l'haptique (3a, 3b) associé à l'ensemble (9a, 9b) et ont une séquence de sectionnement, chacun des au moins deux haptiques (3a, 3b) ayant respectivement un état comprimé, un état partiellement comprimé et un état non comprimé ; pour chacun des au moins deux haptiques (3a, 3b) et l'ensemble (9a, 9b) associé à l'haptique respectif (3a, 3b), à l'état comprimé, la totalité des ressorts (10a, 10b, 10c) étant adaptée pour déformer l'haptique (3a, 3b) en direction du corps optique (2), moyennant quoi au moins le premier ressort (10a) de la séquence de sectionnement est allongé à partir de sa position de repos, et l'haptique (3a, 3b) pouvant être amené par sectionnement successif des ressorts (10a, 10b, 10c) dans la séquence de sectionnement d'abord à l'état partiellement comprimé, dans lequel la totalité des ressorts non sectionnés (10b, 10c) est adaptée pour déformer l'haptique (3a, 3b) en direction du corps optique (2), et au moins celui des ressorts (10b, 10c) qui n'est pas sectionné et qui a le numéro d'ordre le plus bas de la séquence de sectionnement est allongé à partir de sa position de repos, et enfin à l'état non comprimé dans lequel tous les ressorts (10a, 10b, 10c) sont sectionnés.

6. Lentille intraoculaire avec un corps optique (2), au moins deux haptiques (3a, 3b) et, pour au moins deux des haptiques (3a, 3b), respectivement un ensemble (9a, 9b) qui présente une pluralité de ressorts (10a, 10b, 10c) qui sont fixés respectivement au corps optique (2) et à l'haptique (3a, 3b) associé à l'ensemble (9a, 9b), chacun des au moins deux haptiques (3a, 3b) ayant respectivement un état comprimé, un état partiellement comprimé et un état non comprimé ; pour chacun des au moins deux haptiques (3a, 3b) et l'ensemble (9a, 9b) associé à l'haptique respectif (3a, 3b), à l'état comprimé, tous les ressorts (10a, 10b, 10c) étant dans un état de ressort comprimé, et l'haptique (3a, 3b) pouvant être amenée, en amenant successivement les ressorts (10a, 10b, 10c) à un état de ressort non comprimé, d'abord à l'état partiellement comprimé dans lequel au moins un des ressorts (10a, 10b, 10c) se trouve à l'état de ressort comprimé et au moins un des ressorts (10a, 10b, 10c) se trouve à l'état de ressort non comprimé, et enfin à l'état non comprimé, dans lequel tous les ressorts (10a, 10b, 10c) se trouvent à l'état non comprimé.

7. Lentille intraoculaire selon la revendication 6, dans laquelle les ressorts (10a, 10b, 10c) présentent un matériau à mémoire de forme, de telle sorte que chacun des ressorts (10a, 10b, 10c) peut être amené à l'état de ressort non comprimé par un chauffage du matériau à mémoire de forme, ou dans laquelle la lentille intraoculaire (1) présente respectivement un dispositif de maintien pour chacun des ressorts (10a, 10b, 10c) qui est adapté pour maintenir le ressort (10a, 10b, 10c) associé au dispositif de maintien à l'état de ressort comprimé, le ressort (10a, 10b, 10c) associé au dispositif de maintien pouvant être amené à l'état de ressort non comprimé par une ouverture du dispositif de maintien.

8. Lentille intraoculaire selon l'une quelconque des revendications 1 à 7, dans laquelle les au moins deux des haptiques (3a, 3b) sont en forme de C ou en forme de J.

9. Lentille intraoculaire selon l'une quelconque des revendications 1 à 8, dans laquelle la lentille intraoculaire (1) présente trois ou quatre des haptiques (3a, 3b) et, pour chacun des haptiques (3a, 3b), un des ensembles (9a, 9b), notamment la lentille intraoculaire (1) présente seulement deux, seulement trois ou seulement quatre des haptiques (3a, 3b) et, pour chacun des haptiques (3a, 3b), un des ensembles (9a, 9b).

10. Lentille intraoculaire selon l'une quelconque des revendications 1 à 9, dans laquelle le corps optique (2) est un corps optique torique.
